(19) Europäisches Patentamt / European Patent Office / Office européen des brevets

(11) **EP 4 252 737 A1**

(12) **EUROPÄISCHE PATENTANMELDUNG**

(43) Veröffentlichungstag:
04.10.2023 Patentblatt 2023/40

(21) Anmeldenummer: **22165582.2**

(22) Anmeldetag: **30.03.2022**

(51) Internationale Patentklassifikation (IPC):
*A61K 8/02* (2006.01)   *A61K 8/25* (2006.01)
*A61K 8/44* (2006.01)   *A61Q 17/04* (2006.01)

(52) Gemeinsame Patentklassifikation (CPC):
**A61K 8/0266; A61K 8/25; A61K 8/44; A61Q 17/04**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **STADA ARZNEIMITTEL AG**
**61118 Bad Vilbel (DE)**

(72) Erfinder:
• **Beyer, Monika**
**61130 Nidderau (DE)**
• **Ost, Martin**
**63517 Rodenbach (DE)**
• **Heppner, Andrea**
**61197 Florstadt (DE)**

(74) Vertreter: **Hamm&Wittkopp Patentanwälte PartmbB**
**Jungfernstieg 38**
**20354 Hamburg (DE)**

(54) **LICHTSCHUTZZUSAMMENSETZUNG ZUM SCHUTZ DER HAUT VOR SICHTBAREM LICHT**

(57) Die vorliegende Erfindung betrifft eine Lichtschutzzusammensetzung zum Schutz der Haut vor UV-Strahlung und sichtbarem Licht, die neben mindestens einem UV-Filter eine Kombination aus mindestens einem Kompositpartikel, mindestens einem Antioxidationsmittel und mindestens einem Glas enthält.

EP 4 252 737 A1

**Beschreibung**

Die vorliegende Erfindung betrifft eine Lichtschutzzusammensetzung zum Schutz der Haut vor UV-Strahlung und sichtbarem Licht, die neben mindestens einem UV-Filter eine Kombination aus mindestens einem Kompositpartikel, mindestens einem Antioxidationsmittel und mindestens einem Glas enthält.

Die schädigende Wirkung des ultravioletten Teils der Sonnenstrahlung (280 nm-400 nm) auf die Haut ist seit Langem bekannt. Zum Schutz der Haut sowohl vor UVA-Strahlung als auch vor UVB-Strahlung sind zahlreiche chemische und physikalische Filtersubstanzen entwickelt worden, die durch Auftragen auf die Haut in Form pharmazeutischer oder kosmetischer Zusammensetzungen die Wirkung der Sonnenstrahlen im UVA- und UVB-Bereich abschwächen. Die UV-Strahlung macht ca. 5 % des Sonnenlichtes aus. UVB-Strahlung verursacht hauptsächlich Sonnenbrand und DNA-Schäden und dringt bis in die Basalschicht der Haut vor. UVA-Strahlung verursacht hauptsächlich eine DNA-Oxidation und Immunsuppression und dringt bis zur Dermis vor.

Auch die schädigende Wirkung des infraroten Teils des Sonnenlichtes (760 nm-1400 nm) auf die Haut ist bekannt. Der IR Anteil entspricht ca. 45 % des Sonnenlichtes und dringt tief in die Haut ein - bis zur Hypodermis. Infrarotlicht aktiviert die Bildung von Matrixmetalloproteinase-1 (MMP-1), eine Kollagenase, in den Fibroblasten der menschlichen Haut und führt damit, vermittelt über die Aktivierung des MAPK-Signalweges, zu einem Abbau des Kollagens und ist damit für die vorzeitige Hautalterung mitverantwortlich.

Neuere Untersuchungen haben ergeben, dass auch das sichtbare Licht (400 nm-760 nm) einen schädigenden Einfluss auf die Haut haben kann. Sichtbares Licht ist nicht nur wesentlicher Bestandteil des Sonnenlichts und existentiell für das Leben auf der Erde, sondern auch Teil des künstlichen Lichts. Es umfasst ungefähr 50 % des totalen Sonnenspektrums und dringt tief in die Haut ein. Ungefähr 20 % erreicht die Hypodermis.

Dupont et al. beschreibt im International Journal of Cosmetic Science, 1-9, 2013, die durch das Sonnenlicht entstandenen Hautschäden. Durch sichtbares Licht können inflammatorische Cytokine erzeugt werden wie IL-1, IL-6, IL-8 und Matrix abbauende Enzyme wie MMP-1 und MMP-9 (in vitro), freie Radikale (in vivo), Bildung von oxidierten DNA-Basen, Pigmentfärbung bei Personen mit dunklerer Haut.

Sichtbares Licht kann in der Haut eine sogenannte Hyperpigmentierung auslösen.

Duteil et al. beschreibt beispielsweise in Pigment Cell Melanoma Res. 27, 822-826, 2014, den Einfluss von sichtbarem Licht, insbesondere aus dem höherenergetischen Bereich, auf die Haut und beobachtet eine dosisabhängige Hyperpigmentierung, die 3 Monate anhält.

Darüber hinaus kann, wie bereits für UVA- und IR-Strahlung bekannt, auch sichtbares Licht zu vorzeitiger Hautalterung führen, da es zu einer Aktivierung der Matrixmetalloproteinasen, insbesondere MMP-1 führt. In EP 1 591 105 wurde gezeigt, dass IR-Strahlung MMP-1 aufreguliert und somit zur vorzeitigen Hautalterung führt. Dort wurden Antioxidantien als Lösung vorgeschlagen, die in den Aufregulationsprozess der MMP-1 eingreifen.

Zastrow et al. veröffentlicht in Skin Pharmacol. Physiol. 22, 31-44, 2009, die mittels Elektronenspinresonanzspektroskopie bestimmten, durch UV- und sichtbares Licht entstandenen freien Radikale (hauptsächlich ROS: reactive oxygen species). Demnach wird 50 % der totalen oxidativen Hautbelastung durch sichtbares Licht verursacht.

Die Auswirkungen der UV-Strahlung auf die Haut können durch auf die Haut aufgetragene UV-Filter verringert werden. Dazu werden Lichtschutzzusammensetzungen mit UV-Filtern auf die Haut aufgetragen.

Es bestand somit ein Bedürfnis nach Lichtschutzzubereitungen, die die Haut nicht nur vor einer Schädigung durch UV-Strahlung und IR-Strahlung schützen, sondern auch vor sichtbarem Licht bzw. seinen negativen Folgen für die Haut.

Aufgabe der vorliegenden Erfindung ist es somit, herkömmliche Lichtschutzzubereitungen, die wenigstens einen UV-Filter enthalten, zu verbessern und eine Möglichkeit zum Schutz der Haut vor den Folgen des sichtbaren Lichtes bereitzustellen.

Es wurde überraschend gefunden, dass Lichtschutzzusammensetzungen, umfassend mindestens einen UV-Filter sowie eine Kombination aus

- mindestens einem Kompositpartikel
- mindestens einem Antioxidationsmittel und
- mindestens einem Glas

die Haut vor UV-Strahlung, IR-Strahlung und sichtbarem Licht bzw. deren negativen Folgen schützen.

Der erfindungsgemäße Kompositpartikel enthält einen Substratkern aus natürlichem Mica (Glimmer), synthetischem Mica (INCI: Synthetic Fluorphlogopite), Bismuth Oxychloride, Siliziumdioxid, Aluminiumoxid, Aluminimborosilicate, Glas, Bornitrid oder Mischungen davon, auf dem mindestens eine Metalloxidschicht aufgebracht ist.

Der Substratkern umfasst bevorzugt Mica, ausgewählt aus natürlichem Mica (Glimmer), synthetischem Mica (INCI: Synthetic Fluorphlogopite) und einer Mischung davon. In einer besonders bevorzugten Ausführungsform umfasst er natürliches Mica.

In einer weiteren besonders bevorzugten Ausführungsform der Erfindung besteht der Substratkern aus natürlichem Mica, synthetischem Mica oder einer Mischung davon. In einer ganz besonders bevorzugten Ausführungsform der Erfindung besteht der Substratkern aus natürlichem Mica.

Wenn die Zusammensetzung mehr als einen Kompositpartikel enthält, weisen diese voneinander verschiedene Substratkerne und/oder voneinander verschiedene Beschichtungen auf.

In einer bevorzugten Ausführungsform der Erfindung liegt der Substratkern in Form eines dünnen Plättchens vor.

In einer weiteren bevorzugten Ausführungsform der Erfindung liegt der Substratkern in Form eines semitransparenten oder transparenten dünnen Plättchens vor.

Der erfindungsgemäße Kompositpartikel umfasst

- einen Substratkern
- eine erste Beschichtung auf dem Substratkern aus einem ersten Metalloxid
- gegebenenfalls weitere Schichten auf der ersten Beschichtung aus weiteren Metalloxiden, insbesondere einer zweiten Schicht aus einem zweiten Metalloxid.

In einer bevorzugten Ausführungsform der Erfindung umfasst der Kompositpartikel

- einen Substratkern aus Mica
- eine erste Beschichtung auf dem Substratkern aus einem ersten Metalloxid
- gegebenenfalls weitere Schichten auf der ersten Beschichtung aus weiteren Metalloxiden, insbesondere einer zweiten Schicht aus einem zweiten Metalloxid.

Vorteilhafte erste, zweite und weitere Metalloxide sind zum Beispiel Titan-(IV)-oxid, Eisenoxid, Aluminiumoxid, Siliciumdioxid, Zirkoniumoxid, Cobaltoxid, Zinn-(II)-oxid, Zinn-(IV)-oxid, Nickeloxid und Mischungen dieser Oxide. Titan-(IV)-oxid und Zinn-(IV)-oxid sind bevorzugt. Bevorzugt sind die Metalloxide der aneinandergrenzenden Schichten verschieden voneinander. Schichten aus gleichen Metalloxiden können alternierend angeordnet sein. In einer bevorzugten Ausführungsform der Erfindung mit zwei Metalloxidschichten ist eine aus Zinn-(IV)-oxid und die andere aus Titan-(IV)-oxid.

In einer bevorzugten Ausführungsform der Erfindung ist der Kompositpartikel aus Mica, Titanium Dioxide und Tin Oxide aufgebaut, wobei der Substratkern aus Mica mit Schichten aus Titanium Dioxide (INCI-Nomenklatur für Titan-(IV)-oxid) und Tin Oxide (INCI-Nomenklatur für Zinn-(IV)-oxid) umgeben ist. Bevorzugt liegen alternierende Schichten aus Titanium Dioxide und Tin Oxide vor. Das Titanium Dioxide kann in verschiedenen Modifikationen vorliegen, z.B. in der Anatas Modifikation oder der Rutil Modifikation. Um Titandioxidschichten in Rutilform bereitzustellen, wird der Substratkern aus Mica zunächst mit einer dünnen Schicht aus Zinn-(IV)-oxid vorbelegt, auf die dann Titan-(IV)-oxid aufgebracht wird, das nach anschließendem Tempern in der Rutilform vorliegt. In einer bevorzugten Ausführungsform ist deshalb der Substratkern aus Mica mit einer Schicht aus Zinn-(IV)-oxid beschichtet, auf der sich eine Schicht aus Titanium Dioxide, bevorzugt in Rutilform, befindet.

Der bevorzugt eingesetzte Kompositpartikel hat die INCI-Bezeichnung "Titanium Dioxide (and) Mica (and) Tin Oxide".

Der Kompositpartikel im Sinne der Erfindung besteht vorzugsweise ausschließlich aus anorganischen Materialien, ist also vorzugsweise ein anorganischer Kompositpartikel.

Gemäß einer bevorzugten Ausführungsform weist der Kompositpartikel nebst dem Substratkern, der vorzugsweise aus Mica besteht, ausschließlich eine oder mehrere Metalloxidschichten auf, so dass der Kompositpartikel gemäß dieser Ausführungsform frei von Beschichtungen aus Nicht-Metalloxiden ist, insbesondere frei von Beschichtungen aus Stearoyl Glutamate, Disodium Stearolyglutamate, Silicone, Dimethicone, Methicone, Simethicone, Stearic Acid, Cetyl Phosphate, Polysaccharide, Polyacrylate, Polyether und Polyethersilanen.

Gemäß einer bevorzugten Ausführungsform der Erfindung beruht die Wirkung des Kompositpartikels auf Lichtstreuung, Absorption, Reflektion und/oder auf Interferenz des Lichtes an den dünnen, hochbrechenden Metalloxidschichten. Durch unterschiedliche Schichtdicken kann z. B. der Weglängenunterschied zwischen dem an einer äußeren Metalloxidschicht reflektiertem Strahlungsanteil und dem in die äußere Schicht zunächst transmittierten Strahlungsanteil, der dann an der Substratoberfläche bzw. der Metalloxidoberfläche der inneren Schicht reflektiert wird, variiert werden.

Der Kompositpartikel, insbesondere der bevorzugt verwendete aus Mica, Titanium Dioxide und Tin Oxide, reflektiert sichtbares Licht.

Der Kompositpartikel ist kommerziell beispielsweise von der Firma BASF unter den Bezeichnungen Cellini, Cloisonné, Duochrome, Flamenco, Gemtone oder Timica oder beispielsweise von der Firma Merck unter den Bezeichnungen Timiron, Colorona, Xirona, Ronaflair, Ronaflair Balance Red, Ronaflair Balance Gold, Ronaflair Balance Blue oder Ronaflair Balance Green oder beispielsweise von der Firma Kobo unter den Bezeichnungen KTZ, Kobopearl Perpetual oder K-Ray oder beispielsweise von der Firma Eckart unter den Bezeichnungen Syncrystal erhältlich.

Der Kompositpartikel weist bevorzugt eine mit Laserbeugung bestimmte Partikelgröße von 1 $\mu$m bis 200 $\mu$m, besonders bevorzugt von 1 $\mu$m bis 100 $\mu$m und ganz besonders bevorzugt von weniger als 15 $\mu$m (80 % innerhalb dieses Bereichs) auf. In einer Ausführungsform der Erfindung beträgt die mit Laserbeugung bestimmte Teilchengröße 1 bis 15 $\mu$m.

Der Kompositpartikel, der aufgebaut ist aus Mica, Titanium Dioxide und Tin Oxide (INCI: Titanium Dioxide (and) Mica (and) Tin Oxide) weist üblicherweise eine mit Laserbeugung bestimmte Teilchengröße von weniger als 15 $\mu$m (80% innerhalb dieses Bereichs), bevorzugt zwischen 4 bis 10 $\mu$m auf und eine mit Laserbeugung bestimmte Teilchengrößenverteilung von D50 im Bereich von 4 bis 10 $\mu$m, bevorzugt im Bereich von 6,0 bis 8,5 $\mu$m.

Der Kompositpartikel kann nach dem Fachmann geläufigen Verfahren hergestellt werden, wie z. B. dem Zerkleinern und Klassieren von mit Titandioxid beschichteten Glimmerplättchen (natürliches Mica), die gegebenenfalls mit Zinn-(IV)-oxid vorbeschichtet sind, der Hydrolyse von Titansalzlösungen in wässriger Glimmersuspension, wobei auch hier der Glimmer mit Zinn-(IV)-oxid vorbeschichtet sein kann, oder der Gasphasenabscheidung. Beispielhafte Herstellungsverfahren sind in US 4,086,100, US 4,038,099, EP 0 271 767 und US 4,867,794 beschrieben.

Der Kompositpartikel wird üblicherweise in kosmetischen Formulierungen als Effektpigment oder als funktioneller Füllstoff verwendet.

Glanz- oder Effektpigment wird in vielen Bereichen der Technik eingesetzt, insbesondere in der dekorativen Beschichtung, in Farben, Lacken, Druckfarben sowie in kosmetischen Formulierungen. Sie bestehen in der Regel aus plättchenförmigen Trägern, die mit dünnen Metalloxidschichten beschichtet sind. Die optische Wirkung dieser Pigmente beruht auf der gerichteten Reflexion von Licht an den vorwiegend parallel ausgerichteten Plättchen. Dabei entstehen durch Reflexion des Lichtes an den Grenzflächen von Schichten mit unterschiedlichem Brechungsindex Interferenzfarben.

Funktioneller Füllstoff wird in einer Reihe von kosmetischen Produkten verwendet, um wichtige sensorische und optische Eigenschaften einzustellen. Während Farbpigmente, Farbstoffe, Farbstoffe und Parfüme der Kosmetik Fülle und Charakter verleihen, stimmen funktionelle Füllstoffpigmente subtile Eigenschaften wie Viskosität, Härte, Verlauf, Volumen, Hautgefühl, Transparenz und Weichzeichner-Effekte ab, die von entscheidender Bedeutung sind für die haptischen Eigenschaften eines kosmetischen Produktes. Da funktioneller Füllstoff in der Regel nicht die vorherrschenden Eigenschaften von Kosmetika wie Farbe oder Geruch bestimmt, handelt es sich in der Regel um geruchloses, weißes Puder, das angenehmes Hautgefühl bewirkt. Funktioneller Füllstoff trägt durch sein lichtstreuendes und mattierendes Verhalten maßgeblich zum optischen Erscheinungsbild von Kosmetika auf der Haut bei. Einfallendes Licht wird von den Füllstoffteilchen mehr oder weniger in alle Richtungen reflektiert; dies wird oft als diffuse Reflexion bezeichnet. Eine hohe Streuung bewirkt häufig ein weißliches Aussehen und das optische Verbergen der Haut, wohingegen eine geringe Streuung zu einer hohen Transparenz sowohl in der Formulierung als auch auf der Haut führt. Daneben können auch Interferenzeffekte, insbesondere glanzarme Interferenzeffekte auftreten, die zum Beispiel zur Korrektur des Hauttons beitragen.

Die Kompositpartikel sind in den erfindungsgemäßen Lichtschutzzusammensetzungen in einer Menge von 0,01 bis 2 Gew.-%, bevorzugt 0,05 bis 1,5 Gew.-% und besonders bevorzugt 0,1 bis 1 Gew.-% enthalten.

Die Kompositpartikel sind erfindungsgemäß bevorzugt in einer Konzentration enthalten, so dass sie für den Betrachter visuell kaum oder gar nicht wahrnehmbar sind.

Die erfindungsgemäßen Lichtschutzzubereitungen sind bevorzugt keine Schminkprodukte (dekorative Kosmetik).

Das erfindungsgemäß eingesetzte Antioxidationsmittel ist bevorzugt ausgewählt aus der Gruppe bestehend aus: Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), geeignete Phenyl-Propan-Körper, insbesondere Kaffeesäure und deren Derivate, z.B. Ester der Kaffeesäure, Carotinoide (z.B. Lycopin, Lutein), Carotine (z.B. $\alpha$-Carotin, $\beta$-Carotin) und deren Deritvate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Lionleyl-, Cholesteryl- und Glycerylester) sowie deren Salze, Dilaurylthiodipropionat, Distearylthiodiproprionat, Thiodipropionsäure und deren Derivate (Ester, Ether, Peptide, Lipide, Nukleotide, Nukleoside und deren Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigten Fettsäuren und deren Derivate (z.B. $\gamma$-Linolensäure, Linolsäure, Ölsäure), Folsäure und deren Derivate, Alanindiessigsäure, Flavonoide (z.B. Rutin und dessen Derivate, z.B. Troxerutin), Polyphenole, Catechine, Ubichinon und Ubichinol und deren Derivate, Vitamin C und dessen Derivate (z.B. Ascorbylpalmitat, Mg-Ascorbylphosphat, Ascorbylacetat), Tocopherole und deren Derivate (z.B. Vitamin E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4 ), Selen und dessen Derivate (z.B. Selenmethionin), Stilbene und deren Derivate (z.B. Stilbenoxid, Trans-Stilbenoxid) und die Derivate (Salze, Ester, Ether, Zucker, Nukleotide, Nukleoside, Peptide und Liptide) Phytoen, D-Biotin, Coenzym Q10, $\alpha$-Glycosylrutin, Carnitin, Carnosin, natürliche und/oder synthetische Isoflavonoide, Kreatin, Taurin und/oder $\beta$-Alanin sowie 8-Hexadecen-1,16-dicarbonsäure sowie Vitis Vinifera (Grape) seed extract, Haferextrakt, Cichorium intubybus (chicory) leaf extract, Leontopodium Alpinum Extract (Edelweiss), Grünteeextrakt, Curcumin, Silymarin, Apigenin, Resveratrol oder Emblica. Sie können einzeln oder in Kombination mehrerer Antioxidationsmittel eingesetzt werden.

Das Antioxidationsmittel ist in den erfindungsgemäßen Lichtschutzzusammensetzungen in einer Menge von 0,001 bis 20 Gew.-%, bevorzugt 0,01 bis 10 Gew.-%, besonders bevorzugt 0,01 bis 5 Gew.-% und insbesondere 0,05 bis 2 Gew.-% enthalten.

In einer bevorzugten Ausführungsform ist das Antioxidationsmittel ein Peptid, besonders bevorzugt das Dipeptid Carnosine (ß-Alanyl-L-histidin). Carnosine ist aufgebaut aus den Aminosäuren $\beta$-Alanin und L-Histidin. Es kommt natürlich im menschlichen Körper vor. Carnosine ist kommerziell beispielsweise von der Firma Symrise unter der Bezeichnung *Dragosine* erhältlich.

Carnosine ist in den erfindungsgemäßen Lichtschutzzusammensetzungen in einer Menge von 0,001 bis 10 Gew.-%, bevorzugt 0,01 bis 1 Gew.-%, besonders bevorzugt 0,01 bis 0,5 Gew.-% enthalten.

Es können verschiedene Antioxidationsmittel gleichzeitig in den erfindungsgemäßen Lichtschutzzubereitungen eingesetzt werden.

Glas ist ein amorpher, nichtkristalliner Feststoff, welcher durch das Abkühlen und Erstarren einer Schmelze eines anorganischen Stoffgemisches ohne Kristallisation erhalten wird. Man unterscheidet synthetisches und natürliches Glas, wobei natürliches Glas erfindungsgemäß bevorzugt ist.

Gemäß einer bevorzugten Ausführungsform besteht das Glas überwiegend aus Siliziumdioxid.

Erfindungsgemäß eingesetztes synthetisches Glas ist bevorzugt ausgewählt aus der Gruppe bestehend aus: E-, A-, C-, ECR-Glas, Borosilikatglas, Silikatglas, Aluminiumsilikatglas, Fensterglas und Laborglas. Besonders bevorzugt ist das erfindungsgemäß eingesetzte Glas ein E, C, ECR-Glas oder ein Borosilikatglas.

Eine typische Zusammensetzung dieser Gläser ist wie folgt:

Tabelle 1: Gläser

|  | E-Glas | C-Glas | ECR-Glas | Calcium-Natrium Borosilikatglas |
|---|---|---|---|---|
| $SiO_2$ | 50-56 % | 64-70 % | 64-70 % | 52 % |
| $K_2O$ | 0-0,1 % | 0-3 % | 0-3 % | 0,2 % |
| $B_2O_3$ | 5-11 % | 2-7 % | 2-5 % | 8,6 % |
| ZnO | 0-5 % | 0-5 % | 1-5 % | - |
| $Na_2O$ | 0-0,5 % | 10-16 % | 8-13 % | 0,3 % |
| MgO | 0-5 % | 1-5 % | 1-4 % | 1,2 % |
| CaO | 16-25 % | 4-10 % | 3-7 % | 22,5 % |
| $Al_2O_3$ | 12-16 % | 0-5 % | 3-6 % | 14,5 % |
| $TiO_2$ | 0-2 % | - | 0-3 % | - |
| $Fe_2O$ | - | - | - | 0,2 % |

In einer bevorzugten Ausführungsform der Erfindung wird das synthetische Glas in Form von Glasplättchen eingesetzt.

Die Herstellung der erfindungsgemäßen Glasplättchen erfolgt aus der Schmelze nach bekannten Verfahren, wie zum Beispiel dem Rohrblasen oder dem Schleuderverfahren. Besonders bevorzugt werden die Glasplättchen nach dem Schleuderverfahren hergestellt, wie es beispielsweise in EP 0 289 240 oder WO 2005/063637 beschrieben ist.

Die in der Lichtschutzzusammensetzung eingesetzten Glasplättchen aus synthetischem Glas weisen bevorzugt eine elektronenmikroskopisch bestimmte Dicke von < 10 $\mu$m und bevorzugt eine durch Laserbeugung bestimmte Teilchengrößenverteilung D50 von < 500 $\mu$m besonders bevorzugt von < 200 $\mu$m und ganz besonders bevorzugt von < 100 $\mu$m auf.

Das erfindungsgemäß eingesetzte natürliche Glas ist bevorzugt ausgewählt aus der Gruppe bestehend aus Obsidian und Perlite.

Es können verschiedene Gläser gleichzeitig in den erfindungsgemäßen Lichtschutzzubereitungen eingesetzt werden.

Die Glasplättchen aus synthetischem Glas sind in den erfindungsgemäßen Lichtschutzzusammensetzungen in einer Menge von 0,01 bis 5 Gew.-%, bevorzugt 0,01 bis 2 Gew.-%, besonders bevorzugt 0,05 bis 1,5 Gew.-% und insbesondere bevorzugt 0,1 bis 1 Gew.-% enthalten.

Das erfindungsgemäß eingesetzte Glas ist besonders bevorzugt ein hydratisiertes, natürliches Glas, wobei das Glas Perlit ganz besonders bevorzugt ist.

Perlit ist ein natürlich vorkommendes Glas vulkanischen Ursprungs, welches entsteht, wenn Lava schnell abkühlt. Es besitzt einen Wassergehalt von 2-6 Gew.-%. Bei ca. 900 °C erfolgt eine thermische Expansion, d.h. das verdampfende Wasser führt zu einer Aufblähung wodurch aufgeblähte Partikel entstehen, die dann bevorzugt zerkleinert in den Lichtschutzzubereitungen eingesetzt werden können.

Das erfindungsgemäß eingesetzte Perlit ist bevorzugt ein aufgeblähtes und zerkleinertes Perlit mit der INCI-Bezeichnung: Perlite.

Vorzugsweise weist das eingesetzte Perlit mit der INCI Bezeichnung Perlite eine mit Laserbeugung bestimmte mittlere Partikelgröße D50 von etwa 15 bis 30 μm, bevorzugt etwa 20 bis 30 μm, besonders bevorzugt etwa 20 μm auf.

Die Herstellung des erfindungsgemäßen Perlits mit der INCI Bezeichnung Perlite kann zum Beispiel wie in WO 02/11882 beschrieben erfolgen.

Perlit mit der INCI Bezeichnung Perlite ist kommerziell beispielsweise von der Firma Imerys unter den Bezeichnungen *ImerCare® 20P, ImerCare® 25P oder ImerCare® 30P* erhältlich.

Perlite (INCI-Bezeichnung) ist in den erfindungsgemäßen Lichtschutzzusammensetzungen in einer Menge von 0,01 bis 2 Gew.-%, bevorzugt 0,05 bis 1,5 Gew.-%, besonders bevorzugt 0,1 bis 1 Gew.-% enthalten.

Die erfindungsgemäße topisch zu verabreichende Lichtschutzzusammensetzung umfasst des Weiteren wenigstens einen UV-Filter. Er kann ausgewählt sein aus anorganischen und organischen UV-Filtern.

Vorteilhafte anorganische UV-Filter sind Metalloxide, insbesondere Oxide des Titans (amorph oder kristallin in der Rutil- und/oder der Anatasform) oder Zinks, in der INCI Nomenklatur Titanium Dioxide bzw. Zinc Oxide. Wenn anorganische Pigmente enthalten sind, werden diese vorzugsweise in mikronisierter Form eingesetzt, vorzugsweise in Teilchengrößen kleiner 100 μm. Sie sind bevorzugt in Mengen von 1-25 Gew.-%, insbesondere 1-10 Gew.-% enthalten. Die anorganischen Lichtschutzfilter können beschichtet sein, zum Beispiel mit Siliziumdioxid, Siliziumdioxid-Hydrat, Aluminiumoxid, Aluminiumstearat, Stearinsäure, Trimethoxycaprylylsilan, Glycerin, Methicone, Dimethicon, Hydrogen Dimethicon, Simethicon, Polysilicone-15, Alkylsilanen oder Mischungen davon.

Derartige Titan-(IV)-oxid Pigmente sind kommerziell erhältlich beispielsweise von der Firm Tayca unter der Handelsbezeichnung MT100T, von der Firma BASF unter der Handelsbezeichnung Uvinul TiO2, von der Firma Merck unter der Handelsbezeichnung Eusolex T-Avo oder von der Firma DSM Nutritional Products unter der Handelsbezeichnung PARSOL® TX.

Zinkoxid Pigmente sind zum Beispiel von der Firma BASF unter der Handelsbezeichnung Z-Cote HP1 oder von der Firma Symrise unter der Handelsbezeichnung Zinkoxid NDM erhältlich.

Erfindungsgemäß verwendete organische UV-Filter sind öllösliche oder wasserlösliche UV-A- und/oder UV-B-Filter sowie sogenannte Breitbandfilter.

Bevorzugte öllösliche UV-Filter sind:

- Ester der Zimtsäure, beispielsweise gemäß der INCI-Nomenklatur Ethylhexyl Methoxycinnamate (*Neo Heliopan AV, Parsol MCX*) oder Isoamyl p-Methoxycinnamate (*Neo Heliopan E 1000*);

- Benzophenone, beispielsweise gemäß der INCI-Nomenklatur Benzophenone-3 *(Neo Heliopan BB)*, Benzophenone-4 *(Uvinul MS 40)* oder Diethylamino Hydroxybenzoyl Hexyl Benzoate *(Uvinul A Plus)*;

- Triazin-Derivate, beispielsweise gemäß der INCI-Nomenklatur Ethylhexyl Triazone *(Uvinul T150)*, Diethylhexyl Butamido Triazone *(Uvasorb HEB)*; Bis-Ethylhexyloxyphenol Methoxyphenyl Triazine *(Tinosorb S)*; Ethylhexyl Bis-Isopentylbenzoxazolylphenyl Melamine *(Uvasorb K2A)* und/oder Tris-Biphenyl Triazine *(Tinosorb A2B)*;

- Dibenzoylmethan-Derivate, beispielsweise gemäß der INCI-Nomenklatur Butyl Methoxydibenzoylmethane *(Parsol 1789)*;

- Octocrylene *(Neo Heliopan 303)*;

- Salicylsäure-Derivate, beispielsweise gemäß der INCI-Nomenklatur Homosalate *(Neo Heliopan HMS)* und/oder Ethylhexyl Salicylate *(Neo Heliopan OS)*;

- Benzotriazol-Derivate, beispielsweise gemäß der INCI-Nomenklatur Methylene Bis-Benzotriazolyl Tetramethylbutylphenol *(Tinosorb M)* und/oder Drometrizole Trisiloxane *(Mexoryl XL)*;

Öllösliche UV-Filter sind, sofern enthalten, jeweils bevorzugt in Mengen von 0,1-15 Gew.-%, vorzugsweise 0,5-10 Gew.-%, insbesondere 0,5-8 Gew.-% bzw. 1-6 Gew.-%, in den topisch zu verabreichenden Zusammensetzungen enthalten.

Bevorzugte wasserlösliche UV-Filter sind:

- Salze der 2-Phenylbenzimidazol-5-sulfonsäure, beispielsweise gemäß der INCI-Nomenklatur Phenylbenzimidazole Sulfonic Acid *(Neo Heliopan Hydro)*, sowie die Sulfonsäure selbst;

- Sulfonsäurederivate des Dibenzimidazols, wie beispielsweise gemäß der INCI-Nomenklatur Disodium Phenyl Dibenzimidazole Tetrasulfonate *(Neo Heliopan AP)*;

- Campher- und Dicamphersulfonsäurederivate, wie beispielsweise gemäß der INCI-Nomeklatur Benzylidene Camphor Sulfonic Acid *(Mexoryl SL)* und Terephthalylidene Dicamphor Sulfonic Acid *(Mexoryl SX)*;

Auch die wasserlöslichen UV-Filter sind, sofern enthalten, jeweils bevorzugt in Mengen von 0,1-15 Gew.-%, vorzugsweise 0,5-10 Gew.-%, insbesondere 0,5-8 Gew.-% bzw. 1-6 Gew.-%, in den topisch zu verabreichenden Zusammensetzungen enthalten.

In einer bevorzugten Ausführungsform der Erfindung umfassen die erfindungsgemäßen topisch zu verabreichenden Lichtschutzzusammensetzungen einen

- Kompositpartikel, umfassend einen Substratkern aus Mica (INCI: Mica), der mit Schichten aus Zinn-(IV)-oxid (INCI: Tin oxide) und Titan-(IV)-oxid (INCI: Titanium Dioxide) umgeben ist,
- Carnosine und
- Perlite.

Ganz besonders bevorzugt weist der Kompositpartikel, umfassend einen Substratkern aus Mica, der mit Schichten aus Zinn-(IV)-oxid (INCI: Tin oxide) und Titan-(IV)-oxid (INCI: Titanium Dioxide) umgeben ist, eine mit Laserbeugung bestimmte Teilchengröße von 1 bis 15 $\mu$m, bevorzugt zwischen 4 bis 10 $\mu$m auf und einen mit Laserbeugung bestimmten mittleren Teilchendurchmesser D50 im Bereich von zwischen 4 bis 10 $\mu$m, bevorzugt im Bereich von 6,0 bis 8,5 $\mu$m.

Die erfindungsgemäße Lichtschutzzusammensetzung kann weitere übliche Zusatzstoffe, wie z.B. Emulgatoren (o/w, w/o), oberflächenaktive Mittel, Filmbildner, Gelbildner, Verdickungsmittel, Fette, Öle, Wachse, Aktivstoffe/Wirkstoffe, Konservierungsmittel, Lösungsmittel, Emollients, Farbstoffe und/oder Pigmente und Additive wie z. B. Feuchthaltemittel, Parfüme, Entschäumer, Bakterizide, Puffer, Salze, Chelatbildner, Gleitmittel, Treibmittel oder dergleichen umfassen.

Die topisch zu verabreichende Lichtschutzzusammensetzung kann beispielsweise als Emulsion, wie zum Beispiel Öl-in-Wasser Emulsion, Wasser-in-Öl Emulsion, Silikonin-Wasser Emulsion, Wasser-in-Silikon Emulsion, Doppelemulsion, wie zum Beispiel W1/O/W2- oder O1/W/O2-Systeme, multiple Emulsion, wie zum Beispiel W1/O1/W2/O2/W3- oder O1/W1/O2/W2-Systeme, wässrige oder lösungsmittelbasierte Formulierung, Tonic, Shampoo, Conditioner formuliert werden und bevorzugt in Form einer Creme, Salbe, Paste, Lotion, Gel, Hydrodispersionsgel, Aerosol, Spray oder Stift bereitgestellt bzw. auf die Haut aufgetragen werden.

Die beanspruchten Lichtschutzzusammensetzungen werden erfindungsgemäß topisch auf die Haut aufgetragen, um diese vor den schädigenden Einflüssen des sichtbaren Lichtes, insbesondere durch energiereiches sichtbares Licht einer Wellenlänge von 400 bis 500 nm, sogenanntem Blaulicht, wie zum Beispiel Pigmentierung zu schützen. Die Anmelderin hat überraschend gefunden, dass die Haut durch Verwendung der erfindungsgemäßen Lichtschutzzusammensetzungen vor durch sichtbares Licht hervorgerufener Melaninbildung, insbesondere vor Hyperpigmentierung, geschützt wird.

Hyperpigmentierung entsteht durch eine lokale Überproduktion von Melanin, wodurch sich Pigmentflecken bilden. Es handelt sich um eine lokalisierte Hyperaktivität von Melanozyten, die an sich in normaler Anzahl vorliegen. Sie tritt insbesondere an Köperstellen auf, die dem Sonnenlicht ausgesetzt sind. Durch topische Auftragung der erfindungsgemäßen Lichtschutzzusammensetzungen wird die Haut vor Überproduktion von Melanin durch sichtbares Licht geschützt.

Darüber hinaus führt die Einwirkung von sichtbarem Licht auf die Haut zu einer Aktivierung der Bildung von Matrixpetalloproteinasen, insbesondere der MMP-1, die für den Kollagenabbau in der Haut verantwortlich ist.

Durch Auftragen der erfindungsgemäßen Lichtschutzzubereitung auf die Haut wird somit auch der durch sichtbares Licht hervorgerufenen Aufregulation der MMP-1 entgegengewirkt.

Ein weiterer Gegenstand der vorliegenden Erfindung ist folglich ein Stoffgemisch, d. h. die erfindungsgemäße Lichtschutzzubereitung, umfassend mindestens einen UV-Filter sowie eine Kombination aus

- mindestens einem Kompositpartikel,
- mindestens einem Antioxidationsmittel und
- mindestens einem Glas

zur topischen Anwendung in einem Verfahren zum Schutz der Haut vor Schädigung durch sichtbares Licht, wobei gemäß einer bevorzugten Ausführungsform die Schädigung durch sichtbares Licht durch Aktivierung der Bildung von MMP-1 hervorgerufen wird und wobei gemäß einer besonders bevorzugten Ausführungsform diese Schädigung die vorzeitige Hautalterung ist und wobei gemäß einer weiteren Ausführungsform die Schädigung durch sichtbares Licht Hyperpigmentierung ist.

Eine bevorzugte Ausführungsform der Erfindung ist die erfindungsgemäße Lichtschutzzubereitung, umfassend mindestens einen UV-Filter sowie eine Kombination aus

- einem Kompositpartikel, umfassend einen Substratkern aus Mica (INCI: Mica), der mit Schichten aus Zinn-(IV)-oxid (INCI: Tin Oxide) und Titan-(IV)-oxid (INCI: Titanium Dioxide) umgeben ist,
- Carnosine und
- Perlite (INCI-Bezeichnung)

zur topischen Anwendung in einem Verfahren zum Schutz der Haut vor Schädigung durch sichtbares Licht, wobei gemäß einer bevorzugten Ausführungsform die Schädigung durch sichtbares Licht durch Aktivierung der Bildung von MMP-1 hervorgerufen wird und wobei gemäß einer besonders bevorzugten Ausführungsform diese Schädigung die vorzeitige Hautalterung ist und wobei gemäß einer weiteren Ausführungsform die Schädigung durch sichtbares Licht Hyperpigmentierung ist.

Die folgenden Vergleichsversuche belegen den durch die erfindungsgemäßen Lichtschutzzubereitungen bereitgestellten Effekt:

Vergleichsversuche:

Es wurde die Transmission von sechs Lichtschutzzusammensetzungen A bis F sowie die Auswirkung der topischen Auftragung von sechs Lichtschutzzusammensetzungen A bis F auf die Melaninbildung in der Haut untersucht. Von den untersuchten Lichtschutzzusammensetzungen A bis F, sind A, C und E erfindungsgemäß und B, D und F stellen die jeweilige Vergleichsrezeptur dar (Mengenangaben in Gew.-%). Bei den Lichtschutzzusammensetzungen A, B, C, und D handelt es sich um Lotionen in Form von Hydrodispersionsgelen. Die Lichtschutzzusammensetzungen E und F wurden als Sprays formuliert. x in Tabelle 2 bedeutet, dass der Zusatzstoff bzw. der UV-Filter in der Formulierung enthalten ist:

Tabelle 2: Lichtschutzzusammensetzungen

| INCI-Nomenklatur | A | B | C | D | E | F |
|---|---|---|---|---|---|---|
| Acrylates Copolymer | × | × | | | | |
| Acrylatec/C 10-30 Alkyl Acrylate Crosspolymer | | | × | × | × | × |
| Xanthan Gum | × | × | × | × | | |
| Galaktoarabinan | | | | | × | × |
| Ubiquinone | × | × | × | × | × | × |
| Polyester-7 | | | | | × | × |
| Sodium Acryloyldimethyltaurate/VP Crosspolymer | × | × | | | | |
| Disodium EDTA | × | × | × | × | | |
| Pentylene Glycol | × | × | × | × | × | × |
| Diisopropyl Adipate | × | × | × | × | × | × |
| Panthenol | | | | | × | × |
| Tromethamine | × | × | × | × | | |

(fortgesetzt)

| INCI-Nomenklatur | A | B | C | D | E | F |
|---|---|---|---|---|---|---|
| Diisopropyl Sebacate | | | | | × | × |
| Neopentyl Glycol Diheptanoate | | | | | × | × |
| Glycerin | × | × | × | × | × | × |
| Phenylbenzimidazole Sulfonic Acid | × | × | × | × | | |
| Ethylhexyl Methoxycrylene | × | × | × | × | | |
| Isoamyl p-Methoxycinnamate | | | | | × | × |
| C12-15 Alkyl Benzoate | × | × | × | × | × | × |
| Ethylhexyl Salicylate | | | × | × | | |
| Ethylhexyl Triazone | | | | | × | × |
| Diethylhexyl Butamido Triazone | × | × | × | × | × | × |
| Diethylamino Hydroxybenzoyl Hexyl Benzoate | × | × | | | × | × |
| Butyl Methoxydibenzoylmethane | × | × | × | × | | |
| Bis-Ethylhexyloxyphenol Methoxyphenyl Triazine | × | × | × | × | × | × |
| Ascorbyl Tetraisopalmitate, | × | × | × | × | × | × |
| Titanium Dioxide (nano), Silica, Dimethicone | × | × | | | | |
| Perlite, (Titanium Dioxide (and) Mica (and) Tin Oxide), Carnosine | 0,8 | | 0,8 | | 0,8 | |
| Isostearic Acid | × | × | × | × | × | × |
| Tocopherol | × | × | × | × | × | × |
| Tocopheryl Acetate | × | × | × | × | × | × |
| Alcohol Denat. | × | × | × | × | × | × |
| Vitis Vinifera Seed Extract | × | × | × | × | × | × |
| Aqua | × | × | × | × | × | × |

Vergleichsversuche photometrische Messung der Transmission:

Die Transmission T [%] der Lichtschutzmittel A bis C wurde mit einem Perkin Elmer Lambda 900 Spectrometer im Bereich von 400-800 nm und im HEV-Bereich von 400-500 nm bestimmt. Die Filmdicke der Probe betrug 0,1 mm. Es wurden unverdünnte Proben vermessen. Die Messwerte entsprechen dem Mittelwert aus 10 Messungen. Angegeben wird die Schutzwirkung [%], bestimmt nach:

$$\text{Schutzwirkung } [\%] = [1 - T_{Product}/T_{Placebo}] \times 100.$$

Die Proben wurden hinsichtlich der Wirksamkeit der erfindungsgemäßen Kombination untersucht: Bestimmung der Schutzwirkung der Lichtschutzzusammensetzung A im Verhältnis zu B, C im Verhältnis zu D und E im Verhältnis zu F.
Mit den erfindungsgemäßen Lichtschutzzusammensetzungen A, C und E, wurde eine Schutzwirkung im Bereich 400 - 800 nm zwischen 47 % und 56 % und im Bereich von 400 - 500 nm zwischen 51 % und 71 % erreicht, wobei die Schutzwirkung im Bereich 400 - 800 nm von Rezeptur A über B nach C, und im Bereich von 400 - 500 nm von Rezeptur C über A nach B zunahm.

Vorklinische Beurteilung von kosmetischen Formulierungen auf die Melaninbildung nach Bestrahlung mit sichtbarem Licht:

Der schützende Effekt der erfindungsgemäßen Lichtschutzzusammensetzungen vor durch sichtbares Licht hervorgerufener Melaninbildung, insbesondere vor Hyperpigmentierung, wurde in einer vorklinischen Studie zur Melaninbildung in Folge der Bestrahlung mit sichtbarem Licht an menschlichen Hautexplantaten demonstriert.

Dazu wurden Hautexplantate von einem gesunden Probanden des Fitzpatrick-Hauttyps III an 4 aufeinanderfolgenden Tagen mit den untersuchten Lichtschutzzusammensetzungen A bis F in einer Dosierung von 25-30 mg/cm$^2$ behandelt. 24 Stunden nach der ersten Behandlung wurden die untersuchten Lichtschutzzusammensetzungen entfernt und die Hautexplantate einer definierten physiologisch relevanten Dosierung von sichtbarem Licht (480 J/cm2) ausgesetzt (Hydrosun 750, KG1-Filter). 24 Stunden nach der vierten Behandlung wurden die Hautexplantate mittels eine Fontana-Masson Färbung histologisch ausgewertet. In der Fontana-Masson-Färbung wird Melanin als schwarzer Farbniederschlag dargestellt. Der ausgewertete Parameter war der prozentuale Anteil des Melanins pro entsprechende Fläche der Basalschicht und der Stachelzellschicht der Epidermis.

Die Behandlung der Hautexplantate mit den erfindungsgemäßen Lichtschutzmitteln A, C und E führt zu einer Reduktion der Melaninbildung von zwischen 50 % und 71 % während die untersuchten Lichtschutzmittel B, D und F nur zu einer Reduktion der Melaninbildung von zwischen 27 % und 54 % führen. Es ist festzustellen, dass die erfindungsgemäßen Lichtschutzzubereitungen A, C und E jeweils eine deutlich stärkere Reduktion der Melaninbildung bewirken als die entsprechenden Vergleichszusammensetzungen B, D und F, die nicht die erfindungsgemäße Kombination enthalten.

In allen Proben A, C und E zeigte sich durch Verwendung der erfindungsgemäßen Kombination aus Titanium Dioxide, Mica, Tin Oxide, Carnosine und Perlite eine deutliche Verminderung der Transmission der Lichtschutzzubereitung im sichtbaren Strahlenbereich und eine deutliche Reduktion der Melaninbildung gegenüber den jeweiligen Vergleichsrezepturen B, D und F, die von Rezeptur C über E zu A zunahm.

Weitere beispielhafte Formulierungen:

Formulierung 1: Spray

| INCI | Gew. % |
|---|---|
| Alcohol | 80,4 |
| Ethylhexyl Triazone | 5,0 |
| Isoamyl p-Methoxycinnamate | 3,0 |
| Octocrylene | 3,0 |
| Butylmethoxydibenzoylmethane | 0,5 |
| α-Bisabolol nat. | 0,1 |
| DL-α-Tocopherol | 1,0 |
| E-Wasser | 6,3 |
| β-Carotin | 0,1 |
| Perlite, (Titanium Dioxide (and) Mica (and) Tin Oxide), Carnosine | 0,6 |

Formulierungen 2-5: Hydrodispersionsgele

| INCI | Gew. % | | | |
|---|---|---|---|---|
| | Form. 2 | Form. 3 | Form. 4 | Form. 5 |
| Isoamyl-p-Methoxycinnanmate | 5 | - | 12,5 | 2,5 |
| Ethylhexyl Methoxycinnamate | 5 | 5 | 2,5 | 2,5 |
| 4-Methylbenzylidene Camphor | 3 | 3 | 4 | - |
| Butylmethoxydibenzoylmethane | 2 | 1 | - | - |
| Ethylhexyl Triazone | 2 | 2 | 1 | - |
| Octocrylene | - | - | 2,5 | 2,5 |
| Phenylbenzimidazole Sulfonic Acid | - | - | - | 2 |

(fortgesetzt)

| INCI | Gew. % | | | |
|---|---|---|---|---|
| | Form. 2 | Form. 3 | Form. 4 | Form. 5 |
| Disodium PhenylDibenzimidazole Tetrasulfonate | - | - | - | 2 |
| α-Tocopherol | 0,5 | 0,5 | - | - |
| α-Tocopherolacetat | - | - | 1 | 0,25 |
| Bienenwachs | - | - | 1 | - |
| Magnesiumstearat | - | - | 0,5 | - |
| Emblica | 0,5 | - | - | 1,5 |
| Vitamin C | - | - | - | 0,5 |
| Glycerin | 3 | 3 | - | 1,5 |
| Disodium EDTA | 0,1 | 0,1 | 0,1 | 0,05 |
| Pentylene Gycol | - | - | 3 | - |
| Magnesiumsulfat | - | - | 0,5 | - |
| α-Bisabolol | - | - | 0,1 | - |
| Panthenol | 1 | 1,0 | - | 0,5 |
| E-Wasser | 67,35 | 57,7 | 58,0 | 72,1 |
| Acrylates C10-30 Alkyl Acrylate Crosspolymer | 0,4 | 0,4 | 0,5 | 0,5 |
| Octyldodecanol | - | 5 | - | - |
| Isopropylmyristat | - | 5 | - | - |
| Caprylic/Capric Triglyceride | - | 5 | - | - |
| Edelweissextrakt | - | 1 | - | - |
| Grünteeextrakt | - | - | 1 | |
| Natriumhydroxid rein | 0,15 | - | - | 0,7 |
| Alcohol | 9 | 9 | 8 | 9,5 |
| Titanium dixoide | - | - | 3 | - |
| Perlite, (Titanium Dioxide (and) Mica (and) Tin Oxide), Carnosine | 1 | 1,3 | 0,8 | 1,4 |

Formulierungen 6-9: Hydodispersionsgele

| INCI | Gew. % | | | |
|---|---|---|---|---|
| | Form. 6 | Form. 7 | Form. 8 | Form. 9 |
| Ethylhexyl Salicylat | 5,0 | 5,0 | 5,0 | 5,0 |
| Bis-Ethylhexyloxyphenol Methoxyphenol Triazine | 2,0 | 2,0 | 2,0 | 2,0 |
| Butylmethoxydibenzovlmethane | 5,0 | 5,0 | 5,0 | 5,0 |
| Ethylhexyl Triazone | 3,0 | 3,0 | 3,0 | 3,0 |
| Diethylamino Hydoxybenzoyl Hexyl Benzoate | - | 5,0 | 10,0 | 5,0 |
| Phenylbenzimidazole Sulfonic Acid | 4,0 | 4,0 | 4,0 | 4,0 |
| Disodium EDTA | 0,10 | 0,10 | 0,10 | 0,10 |
| Pentylene Gycol | 5,0 | 5,0 | 5,0 | 5,0 |

(fortgesetzt)

| INCI | Gew. % | | | |
|------|--------|--------|--------|--------|
| | Form. 6 | Form. 7 | Form. 8 | Form. 9 |
| Xanthan Gum | 0,5 | 0,5 | 0,5 | 0,5 |
| Aqua | 33,25 | 24,05 | 18,95 | 22,85 |
| Acrylates C10-30 Alkyl Acrylate Crosspolymer | 0,35 | 0,35 | 0,35 | 0,35 |
| Tromethamine (20% ige Lösung) | 13,0 | 13,0 | 13,0 | 13,0 |
| C12-15 Alkylbezoate | 16,0 | 20,0 | 20,0 | 20,0 |
| Titanium dioxide, Trimethoxycaprylylsilane | 9,0 | 9,0 | 9,0 | 9,0 |
| Zinc oxide | - | - | - | 1,0 |
| Alcohol Denat. | 3,0 | 3,0 | 3,0 | 3,0 |
| Perlite, (Titanium Dioxide (and) Mica (and) Tin Oxide), Carnosine | 0,8 | 1 | 1,1 | 1,2 |

Formulierung 10: Lotion

| INCI | Gew. % |
|------|--------|
| Alcohol | 9 |
| Ethylhexyl Triazone | 2 |
| Isoamyl p-Methoxycinnamate | 5 |
| 4-Methylbenzylidene Camphor | 3 |
| Butylmethoxydibenzoylmethane | 1 |
| Polyglyceryl-3 Methylglucose Distearate | 2 |
| Hydrogenated Coco-Glycerides | 0,5 |
| Cetyl Palmitate | 0,5 |
| Cetyl Alcohol | 1,5 |
| Cocoglycerides | 5 |
| Carbomer | 0,2 |
| $\alpha$-Tocopherolacetat | 1 |
| E-Wasser | 62,85 |
| Lutein | 0,5 |
| Glycerin | 5 |
| Xanthan Gum | 0,1 |
| Natriumhydroxid rein | 0,046 |
| Perlite, (Titanium Dioxide (and) Mica (and) Tin Oxide), Carnosine | 0,8 |

Formulierung 11: W/O Creme

| INCI | Gew. % |
|------|--------|
| Alcohol | 6 |
| Ethylhexyl Triazone | 1 |
| Isoamyl p-Methoxycinnamate | 10 |

(fortgesetzt)

| INCI | Gew. % |
|---|---|
| 4-Methylbenzylidene Camphor | 4 |
| Isopropylmyristat | 10 |
| Bienenwachs | 1 |
| Magnesiumstearat | 0,5 |
| α-Tocopherolacetat | 1 |
| E-Wasser | 56,7 |
| Disodium EDTA | 0,1 |
| Pentylene Gycol | 3 |
| Magnesiumsulfat | 0,5 |
| α-Bisabolol | 0,1 |
| Grünteeextrakt | 1 |
| Titanium Dioxide | 3 |
| Perlite, (Titanium Dioxide (and) Mica (and) Tin Oxide), Carnosine | 1,1 |

Formulierung 12: O/W-Emulsion

| INCI | Gew. % |
|---|---|
| Polyglyceryl-, Diisostearate/Polyhydroxvstearate/Sebacate | 4,0 |
| Cera Alba | 2,0 |
| Caprylic/Capric Triglyceride | 10,0 |
| C 12-15 Alkyl Benzoate | 6,0 |
| Ethylhexyl Methoxycinnamate | |
| Diethylamino Hydoxybenzoyl Hexyl Benzoate | 5,0 |
| Bis-Ethylhexyloxyphenol Methoxyphenol Triazine | 2,0 |
| Octocrylene | 5,0 |
| Ethylhexal Salicylate | 5,0 |
| Butylmethoxydibenzoylmethane | 5,0 |
| Titanium Dioxide, Silica, Dimethicone | 4,0 |
| C 12-15 Alkyl Benzoate, Steaalkonium Hectorite (10%ig), Propylene Carbonate | 12,0 |
| Tocopheryl Acetate | 0,5 |
| Butylene Glycol | 7,2 |
| Magnesium Sulfate | 0,5 |
| Perlite, (Titanium Dioxide (and) Mica (and) Tin Oxide), Carnosine | 0,9 |
| Trisodium NTA, Aqua | 0,26 |
| Citric Acid | 0,06 |
| Aqua | 30,58 |

Formulierung 13: O/W-Emulsion

| INCI | Gew. % |
|---|---|
| Cetyl Alcohol | 1,0 |
| Cetearyl Alcohol | 1,0 |
| Ethylhexyl Methoxycinnamate | 10,0 |
| Diethylamino Hydoxybenzoyl Hexyl Benzoate | 7,0 |
| Octyldodecanol | 8,0 |
| Decyl Glucoside | 1,0 |
| Zinc oxide | 0,6 |
| Titanium Dioxide, Aluminium Hydroxide Stearic Acid | 8,0 |
| Methylene Bis-Benzotriazolyl Tetramethylbutylphenol | 7,5 |
| Xanthan Gum | 0,3 |
| Acrylates C10-30 Alkyl Acrylate Crosspolymer | 0,5 |
| Sodium EDTA Disodium | 0,1 |
| Glycerin | 5,0 |
| Panthenol | 1,0 |
| Perlite, (Titanium Dioxide (and) Mica (and) Tin Oxide), Carnosine | 0,9 |
| Alcohol Denat. | 10,0 |
| Sodium Hydroxide | 0,05 |
| Aqua | 38,05 |

**Patentansprüche**

1.  Lichtschutzzusammensetzung zum Schutz der Haut vor UV-Strahlung und sichtbarem Licht, umfassend mindestens einen UV-Filter sowie eine Kombination aus

    - mindestens einem Kompositpartikel
    - mindestens einem Antioxidationsmittel und
    - mindestens einem Glas.

2.  Lichtschutzzusammensetzung nach Anspruch 1,
    **dadurch gekennzeichnet, dass** der Kompositpartikel umfasst:

    - einen Substratkern aus Mica, Bismuth Oxychloride, Siliziumdioxid, Aluminiumoxid, Aluminiumborosilicate, Glas, Bornitrid oder Mischungen davon
    - eine erste Beschichtung auf dem Substratkern aus einem ersten Metalloxid
    - gegebenenfalls weitere Schichten auf der ersten Beschichtung aus weiteren Metalloxiden, insbesondere einer zweiten Schicht aus einem zweiten Metalloxid.

3.  Lichtschutzzusammensetzung nach Anspruch 1 oder 2,
    **dadurch gekennzeichnet, dass** der Kompositpartikel einen Substratkern aus Mica umfasst.

4.  Lichtschutzzusammensetzung nach Anspruch 2 oder 3,
    **dadurch gekennzeichnet, dass** das Mica ausgewählt ist aus natürlichem Mica (Glimmer), synthetischem Mica (INCI: Synthetic Fluorphlogopite) oder einer Mischung davon, bevorzugt natürlichem Mica.

5.  Lichtschutzzusammensetzung nach einem der Ansprüche 2 bis 4,
    **dadurch gekennzeichnet, dass** das erste, zweite und weitere Metalloxid gewählt werden aus Titan-(IV)-oxid, Eisenoxid, Aluminiumoxid, Siliciumdioxid, Zirkoniumoxid, Cobaltoxid, Zinn-(IV)-oxid, Zinn-(II)-oxid, Nickeloxid und Mischungen dieser Oxide, bevorzugt aus Titanium Dioxide (INCI-Nomenklatur für Titan-(IV)-oxid) und Tin Oxide (INCI-Nomenklatur für Zinn-(IV)-oxid), wobei besonders bevorzugt des erste Metalloxid Tin Oxide und das zweite Metalloxid Titanium Dioxide ist.

6. Lichtschutzzusammensetzung nach einem der Ansprüche 1 bis 5,
   **dadurch gekennzeichnet, dass** das Glas ausgewählt ist aus synthetischem Glas, natürlichem Glas oder Mischungen davon.

7. Lichtschutzzusammensetzung nach einem der Ansprüche 1 bis 6,
   **dadurch gekennzeichnet, dass** das Glas Perlite (INCI-Bezeichnung) ist.

8. Lichtschutzzusammensetzung nach einem der Ansprüche 1 bis 7,
   **dadurch gekennzeichnet, dass** der Gehalt an Kompositpartikel in der Lichtschutzzubereitung 0,01 bis 2 Gew.-%, bevorzugt 0,05 bis 1,5 Gew.-%, besonders bevorzugt 0,1 bis 1 Gew.-% beträgt.

9. Lichtschutzzusammensetzung nach einem der Ansprüche 1 bis 8,
   **dadurch gekennzeichnet, dass** der Gehalt an Antioxidationsmittel in der Lichtschutzzubereitung 0,001 bis 20 Gew.-%, bevorzugt 0,01 bis 10 Gew.-%, besonders bevorzugt 0,01 bis 5 Gew.-% und insbesondere 0,05 bis 2 Gew.-%, beträgt.

10. Lichtschutzzusammensetzung nach einem der Ansprüche 1 bis 9,
    **dadurch gekennzeichnet, dass** der Gehalt an Glas in der Lichtschutzzubereitung 0,01 bis 5 Gew.-%, bevorzugt 0,01 bis 2 Gew.-%, besonders bevorzugt 0,05 bis 1,5 Gew.-% und insbesondere 0,1 bis 1 Gew.-% beträgt.

11. Lichtschutzzusammensetzung nach einem der Ansprüche 1 bis 10,
    **dadurch gekennzeichnet, dass** der UV-Filter ausgewählt ist aus Ethylhexyl Methoxycinnamate, Isoamyl p-Methoxycinnamate, Benzophenone-3, Benzophenone-4, Diethylamino Hydroxybenzoyl Hexyl Benzoate, Ethylhexyl Triazone, Diethylhexyl Butamido Triazone, Bis-Ethylhexyloxyphenol Methoxyphenyl Triazine, Ethylhexyl Bis-Isopentylbenzoxazolylphenyl Melamine, Tris-Biphenyl Triazine, Butyl Methoxydibenzoylmethane, Octocrylene, Homosalate, Ethylhexyl Salicylate, Methylene Bis-Benzotriazolyl Tetramethylbutylphenol, Drometrizole Trisiloxane, Phenylbenzimidazole Sulfonic Acid, Disodium Phenyl Dibenzimidazole Tetrasulfonate, Benzylidene Camphor Sulfonic Acid, Terephthalylidene Dicamphor Sulfonic Acid, Titanium Dioxide, Zinc Oxide.

12. Lichtschutzzusammensetzung nach einem der Ansprüche 1 bis 11, **dadurch gekennzeichnet, dass** der UV-Filter Titanium Dioxide ist.

13. Lichtschutzzusammensetzung nach einem der Ansprüche 1 bis 12,
    **dadurch gekennzeichnet, dass** der Kompositpartikel eine mit Laserbeugung bestimmte Teilchengröße im Bereich von 1 $\mu$m bis 200 $\mu$m, besonders bevorzugt von 1 $\mu$m bis 100 $\mu$m und ganz besonders bevorzugt von weniger als 15 $\mu$m (80 % innerhalb dieses Bereichs) aufweisen.

14. Verwendung einer Lichtschutzzusammensetzung nach einem der Ansprüche 1 bis 13 zum Schutz der Haut vor durch sichtbares Licht hervorgerufener Pigmentierung, bevorzugt Hyperpigmentierung.

15. Lichtschutzzusammensetzung nach einem der Ansprüche 1 bis 13 zur topischen Anwendung in einem Verfahren zum Schutz der Haut vor Schädigung durch sichtbares Licht.

16. Lichtschutzzusammensetzung nach Anspruch 15,
    **dadurch gekennzeichnet, dass** die Schädigung durch sichtbares Licht durch Aktivierung der Bildung von Matrixmetalloproteinase-1 hervorgerufen ist.

17. Lichtschutzzusammensetzung nach Anspruch 15 oder 16,
    **dadurch gekennzeichnet, dass** die Schädigung durch sichtbares Licht vorzeitige Hautalterung ist.

18. Lichtschutzzusammensetzung nach Anspruch 15,
    **dadurch gekennzeichnet, dass** die Schädigung durch sichtbares Licht Hyperpigmentierung ist.

**EUROPÄISCHER RECHERCHENBERICHT**

Nummer der Anmeldung

EP 22 16 5582

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | "Experimental Effect Pigments EP-99615 and EP-99616 ED – Darl Kuhn", IP.COM, IP.COM INC., WEST HENRIETTA, NY, US, 11. Februar 2014 (2014-02-11), XP013161007, ISSN: 1533-0001 | 1-13 | INV. A61K8/02 A61K8/25 A61K8/44 A61Q17/04 |
| Y | * Color care shimmering CC cream broad spectrum; Seite 6 * * Sunscreen SPF 30; Seite 44 * | 1-18 | |
| | ----- | | |
| X | US 2011/223218 A1 (JONES STEVEN ALAN [US] ET AL) 15. September 2011 (2011-09-15) | 1-6,9, 10,12,13 | |
| Y | * Beispiel 18 * * Absatz [0005] * | 1-18 | |
| | ----- | | |
| X | US 8 728 502 B2 (SIOSS JAMES [US]; CHIRAYIL THOMAS [US] ET AL.) 20. Mai 2014 (2014-05-20) | 1-7,9-13 | |
| Y | * Elegant Sun protection; Spalten 21-24 * | 1-18 | RECHERCHIERTE SACHGEBIETE (IPC) |
| | ----- | | A61K A61Q |
| X | US 2019/240125 A1 (SUZUKI JUN [JP] ET AL) 8. August 2019 (2019-08-08) | 1-6,8-10 | |
| Y | * Beispiel 1; Tabelle 1 * | 1-18 | |
| | ----- | | |
| Y | DE 10 2019 215111 A1 (BEIERSDORF AG [DE]) 1. April 2021 (2021-04-01) * Absätze [0005] – [0007]; Anspruch 1 * * Absätze [0021], [0022]; Ansprüche 7,9 * | 1-18 | |
| | ----- | | |

Der vorliegende Recherchenbericht wurde für alle Patentansprüche erstellt

| Recherchenort | Abschlußdatum der Recherche | Prüfer |
|---|---|---|
| München | 6. Oktober 2022 | Tullberg, Erik |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 16 5582

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-10-2022

| Im Recherchenbericht angeführtes Patentdokument | Datum der Veröffentlichung | Mitglied(er) der Patentfamilie | Datum der Veröffentlichung |
|---|---|---|---|
| US 2011223218 A1 | 15-09-2011 | KEINE | |
| US 8728502 B2 | 20-05-2014 | BR 112013021767 A2 | 18-10-2016 |
| | | CN 103403102 A | 20-11-2013 |
| | | EP 2686386 A2 | 22-01-2014 |
| | | ES 2637115 T3 | 10-10-2017 |
| | | JP 5881750 B2 | 09-03-2016 |
| | | JP 2014508211 A | 03-04-2014 |
| | | KR 20140011333 A | 28-01-2014 |
| | | US 2012237577 A1 | 20-09-2012 |
| | | WO 2012125789 A2 | 20-09-2012 |
| US 2019240125 A1 | 08-08-2019 | CN 109890351 A | 14-06-2019 |
| | | EP 3532011 A1 | 04-09-2019 |
| | | JP 6995472 B2 | 14-01-2022 |
| | | JP 2018070514 A | 10-05-2018 |
| | | KR 20190067865 A | 17-06-2019 |
| | | US 2019240125 A1 | 08-08-2019 |
| | | WO 2018079455 A1 | 03-05-2018 |
| DE 102019215111 A1 | 01-04-2021 | DE 102019215111 A1 | 01-04-2021 |
| | | EP 4037639 A1 | 10-08-2022 |
| | | WO 2021063609 A1 | 08-04-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1591105 A
- US 4086100 A
- US 4038099 A
- EP 0271767 A
- US 4867794 A
- EP 0289240 A
- WO 2005063637 A
- WO 0211882 A

**In der Beschreibung aufgeführte Nicht-Patentliteratur**

- **DUPONT et al.** *International Journal of Cosmetic Science,* 2013, 1-9
- **DUTEIL et al.** *Pigment Cell Melanoma Res.,* 2014, vol. 27, 822-826
- **ZASTROW et al.** *Skin Pharmacol. Physiol.,* 2009, vol. 22, 31-44